# EUROPEAN PATENT SPECIFICATION

(11) **EP 3 125 760 B1**
(45) Date of publication and mention of the grant of the patent: **30.12.2020**
(21) Application number: 15772590.4
(22) Date of filing: 02.04.2015
(51) Int. Cl.: A61B 5/00, A61B 5/1455, A61B 5/145

(54) **SYSTEM AND METHOD FOR DETERMINING BILIRUBIN LEVELS IN NEWBORN BABIES**
SYSTEM UND VERFAHREN ZUR BESTIMMUNG DER BILIRUBINSPIEGEL BEI NEUGEBORENEN
SYSTÈME ET PROCÉDÉ POUR DÉTERMINER LES NIVEAUX DE BILIRUBINE CHEZ LES NOUVEAU-NÉS

(30) Priority: 02.04.2014 US 201461973885 P
(43) Date of publication of application: 08.02.2017
(73) Proprietor: BiliBaby, LLC, Bryn Mawr, PA 19010 (US)
(72) Inventor: CLEMENTE, Matthew, Carmel IN 46032 (US); STAVIS, George, Dobbs Ferry, NY 10522 (US); MELIKECHI, Noureddine, Dover, DE 19904 (US); STAVIS, Robert, Bryn Mawr, PA 19010 (US)
(74) Representative: Cookson, Barbara Elizabeth
(86) International application number: PCT/US2015/024151
(87) International publication number: WO 2015/153913

(56) References cited:
- WO-A1-2016/047754
- WO-A2-2013/096766
- CN-A- 103 340 604
- US-A1- 2002 095 077
- US-A1- 2013 023 742
- US-A1- 2013 057 865
- US-A1- 2013 273 524
- US-A1- 2013 296 673
- US-A1- 2013 300 919
- US-A1- 2014 018 647
- US-A1- 2014 316 226
- US-A1- 2014 323 832

## Description

### RELATED APPLICATIONS

This application claims priority to U.S. Provisional Patent Application No. 61/973,885, filed April 2, 2014.

### FIELD OF THE INVENTION

The present invention relates generally to a system and method for determining levels of a substance in a patient's body based on the patient's skin coloration. The invention provides a device such as a smartphone coupled to a light source and optical detector that receives data regarding the skin coloration and uses that data to calculate the concentration of the substance in the body.

### BACKGROUND OF THE INVENTION

During the first week of life, most newborns develop a visible yellow coloring of the skin - jaundice - due to an increase in a chemical called bilirubin. Moderate levels of bilirubin are benign, but very high levels - called severe hyperbilirubinemia - can cause a condition called kernicterus, which is a severe and life-long severe form of athetoid cerebral palsy with hearing dysfunction, dental-enamel dysplasia, and intellectual handicaps.

In order to reduce the likelihood of kernicterus, the American Academy of Pediatrics recommends that all infants be evaluated for jaundice with systematic measurement of bilirubin, and treated according to specific algorithms. Measurement of bilirubin levels is most accurately done by chemical analysis of a blood specimen, but hand-held instruments have also been developed to estimate bilirubin levels by optical measurement of subcutaneous skin coloration. Because of prohibitively high cost, such instruments are only practical in a hospital setting, rather than in a doctor's office or for home application. There are no currently available technologies for estimating the bilirubin level at a price level consistent with use in a doctor's office or in the home. Accordingly, it is often necessary for infants to return to the hospital to have the bilirubin level checked.

US 2013/023742 A1 decribes such a self-contained instrument for monitoring bilrubin levels in which light is transmitted through a clamped portion of the subjec's living tissue.

The present invention described herein builds upon the functions of smartphones, tablets, computers, digital cameras connected to computers and other home devices to give parents and clinicians a noninvasive, rapid, and relatively easy to implement tool to monitor bilirubin through changes in the skin color of the infant. The invention further provides an affordable method of estimating bilirubin levels in the home or doctor's office that will simplify and vastly improve the outpatient management of hyperbilirubinemia in babies during the first week at home.

### DESCRIPTION OF PRIOR ART

US 2013/0300919 A1 discloses an ancillary module comprising one or more optical filters, for analysing the skin properties of a person and to be adapted on a smart-phone having a camera and a flash-light.

### SUMMARY OF THE INVENTION

The present invention is defined in the claims and more specifically provides a system and method for determining bilirubin levels in an individual based on subcutaneous skin coloration using a smartphone or other personal device and an attached ancillary apparatus. The device, such as a smartphone or tablet, is capable of storing and running software. The device is also coupled to both a camera and light source to obtain data regarding the skin's subcutaneous coloration. Software is installed on the device to control the light source and calculate bilirubin levels in the individual based on the input received from the camera.

### BRIEF DESCRIPTION OF THE DRAWINGS

**FIG. 1** is a diagram depicting the system architecture of the preferred embodiment.
**FIG. 2A** provides a top perspective view of the ancillary device in a smartphone embodiment having one or more physical filters or light-transmitting pathways as well as safety features and utilizing the smartphone's on-board flash and the on-board camera to capture an image and an ancillary module that has incorporated that prevent misuse;
**FIG. 2B** provides a cross sectional view along section A-A of the ancillary device provided in Fig. 2A;
**FIG. 2C** provides a angled elevational view of the ancillary device provided in Fig. 2A;
**FIG. 2D** provides a top perspective view of the ancillary device in an alternative smartphone embodiment having one or more physical filters or light-transmitting pathways as well as safety features and utilizing the smartphone's on-board flash and the on-board camera to capture an image and an ancillary module that has incorporated that prevent misuse;
**FIG. 2E** provides a cross sectional view along section D-D of the ancillary device provided in Fig. 2D;
**FIG. 2F** provides a angled elevational view of the ancillary device provided in Fig. 2D;
**FIG. 3A** provides a graph disclosing the distribution of the light source on the Nokia Lumia 1020 smartphone showing the emission of light at both the 450 nm and 550 nm wavelengths for use with the present invention;
**FIG. 3B** provides a graph disclosing the distribution of the light source on the iPhone 5s smartphone showing the emission of light at both the 450 nm and 550 nm wavelengths for use with the present invention;
**FIG. 3C** provides a graph disclosing the distribution of the light source on the Samsung Galaxy S4 Mini smartphones showing the emission of light at both the 450 nm and 550 nm wavelengths for use with the present invention;
**FIG. 4A** depicts a side view of an embodiment of the ancillary module disclosed in FIG. 2 attached to the smartphone;
**FIG. 4B** depicts a rear view of a smartphone attached to an embodiment of the ancillary module disclosed in FIGS. 2A-2E;
**FIG. 4C** depicts a detailed cross sectional view of section C-C of the embodiment of the ancillary module disclosed in FIG. 4B;
**FIG. 4D** depicts a detailed cross sectional view of section D-D of the embodiment of the ancillary module disclosed in FIG. 4B;
**FIG. 4E** depicts a detailed view of section E shown in FIG. 4D;
**FIG.5A** is a portion of a flowchart illustrating the functions of the software of the preferred embodiment.
**FIG.5B** is a portion of a flowchart continuing from FIG 5A illustrating the functions of the software of the preferred embodiment.
**FIG.5C** is a portion of a flowchart continuing from FIGS 5A and 5B illustrating the functions of the software of the preferred embodiment.

### DETAILED DESCRIPTION

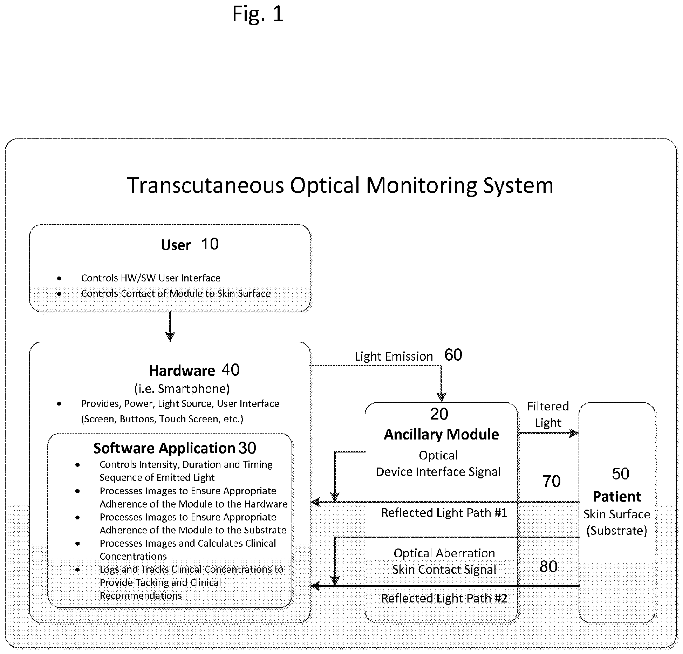

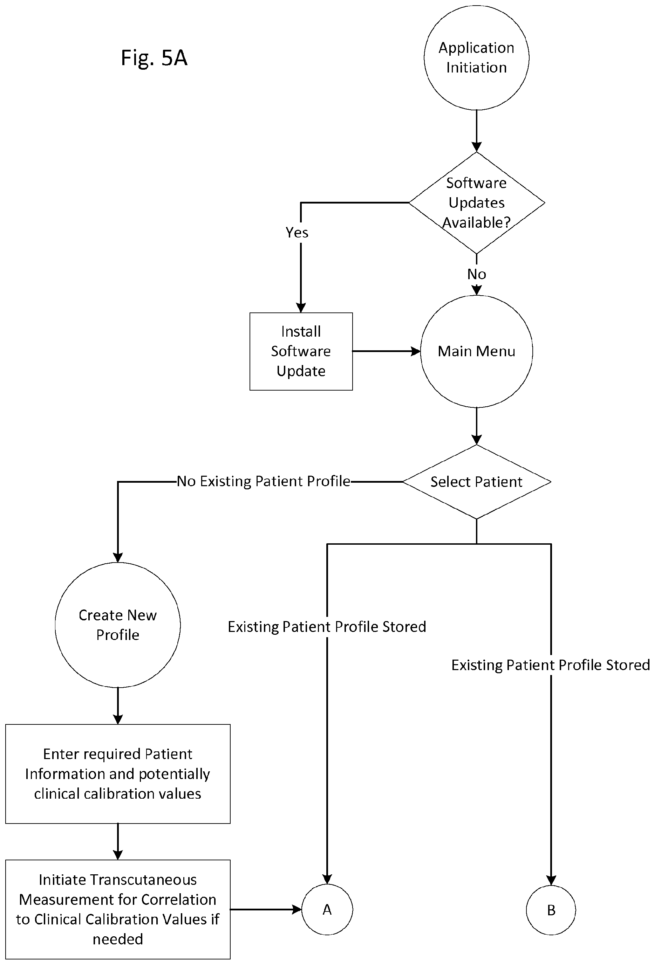

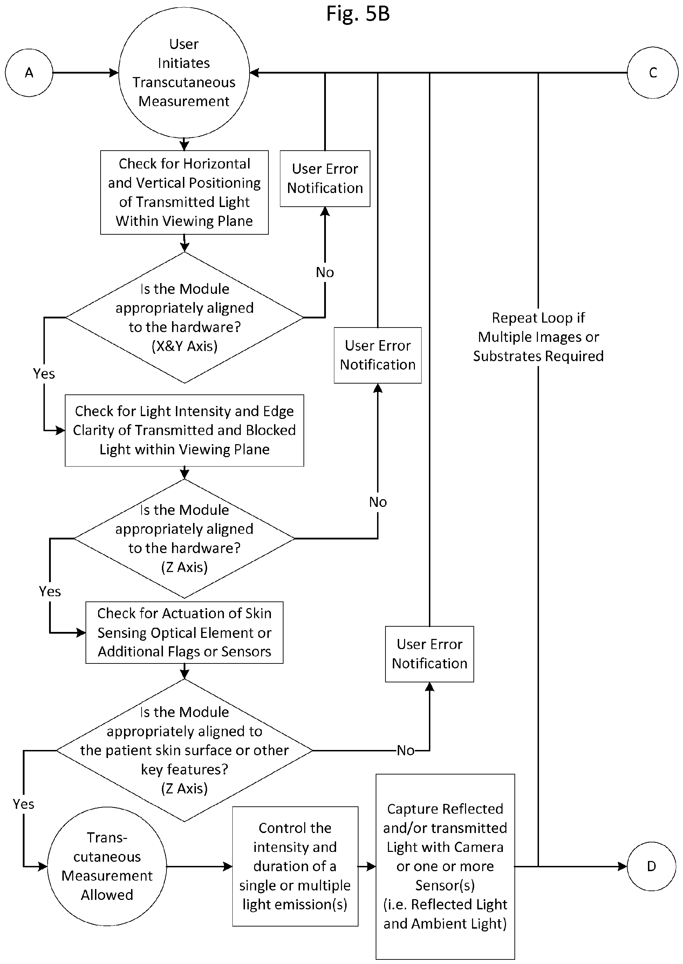

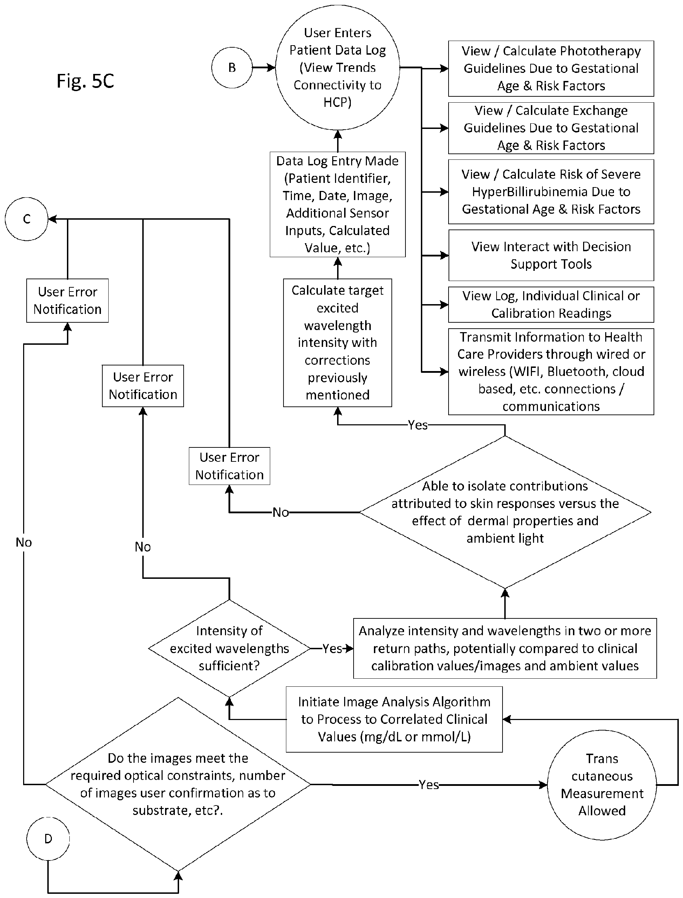

A detailed description will now be given of the invention with reference to the attached Figures 1-5. It should be understood that these Figures are exemplary in nature and in no way serve to limit the scope of the invention.

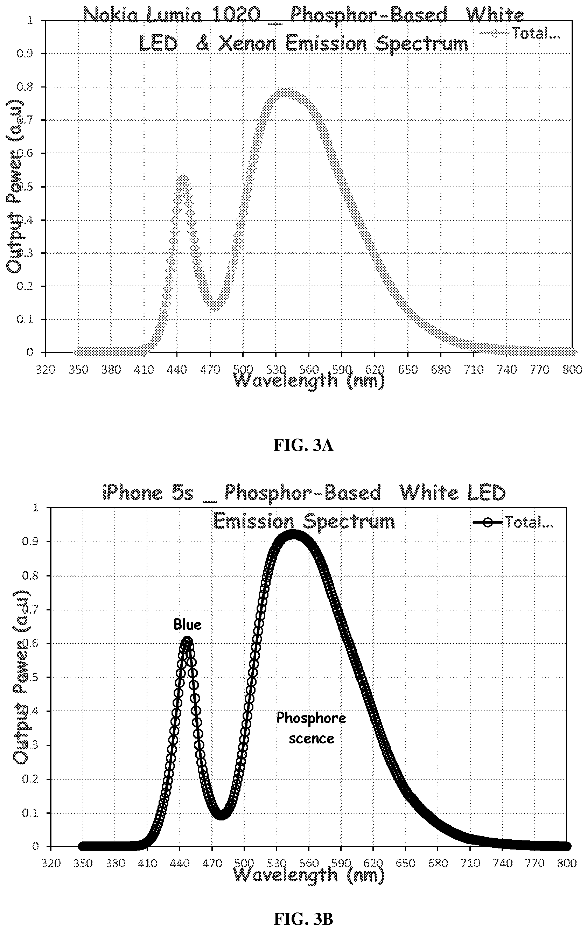

The present invention describes a system and method for measuring the level of bilirubin in a patient based on subcutaneous skin coloration by using a known light source to generate reflected light that is then recorded and analyzed. This system and method utilizes optical imaging methods for obtaining tissue properties based on the emissions of known light sources 60, such as those demonstrated in Figures 3A-3C, and the sensing of light refraction 70 and 80 caused by tissue interactions. The difference in the optical densities found in the various pathways allows the effect of the dermal layers to be removed through analysis to obtain values for the transcutaneous bilirubin in the subcutaneous layer. At the highest level the invention consists of a user 10, a patient 50, hardware 40, such as a smartphone, a software application 30, and an ancillary module 20 as shown in FIG. 1. To use the system, a user connects the ancillary module 20 to the hardware 40, and places the ancillary module onto the patient's skin. In some applications, the user and patient are the same person. The user then, using the software 30 on the hardware 40, emits light 60 from a light source through the ancillary module onto the patient's skin. The emitted light 60 strikes the cutaneous membranes of the user 50. A fraction of the incident energy is reflected at the tissue boundary, and a fraction is transmitted inside the tissue. A portion of the transmitted light is further absorbed and scattered by the tissue. The light distribution in the tissue is affected by the refractive index and absorption scattering characteristics of the tissue. The scattered light is then transmitted through single or multiple optical pathways and detected by a sensor. The optical sensor transmits this data to the software on the device and the software uses this data to calculate the level of bilirubin or other substance in the patient.

The detailed description elaborates the methods by which the ancillary module 20 and the accompanying software application 30 will interface between the hardware 40 and the patient 50. The safety features and methods by which the module and software protects the patient, by reducing the possibility of user error, are also described.

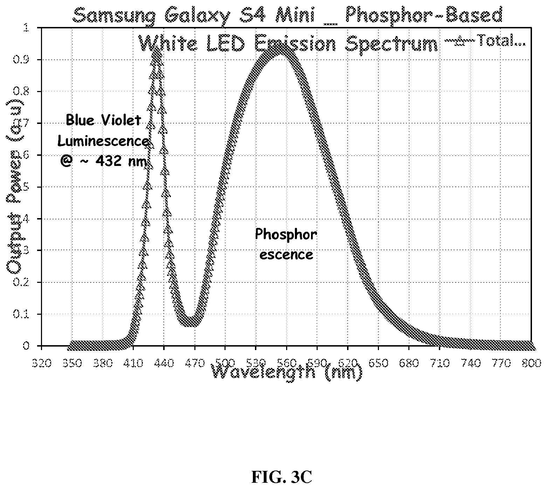

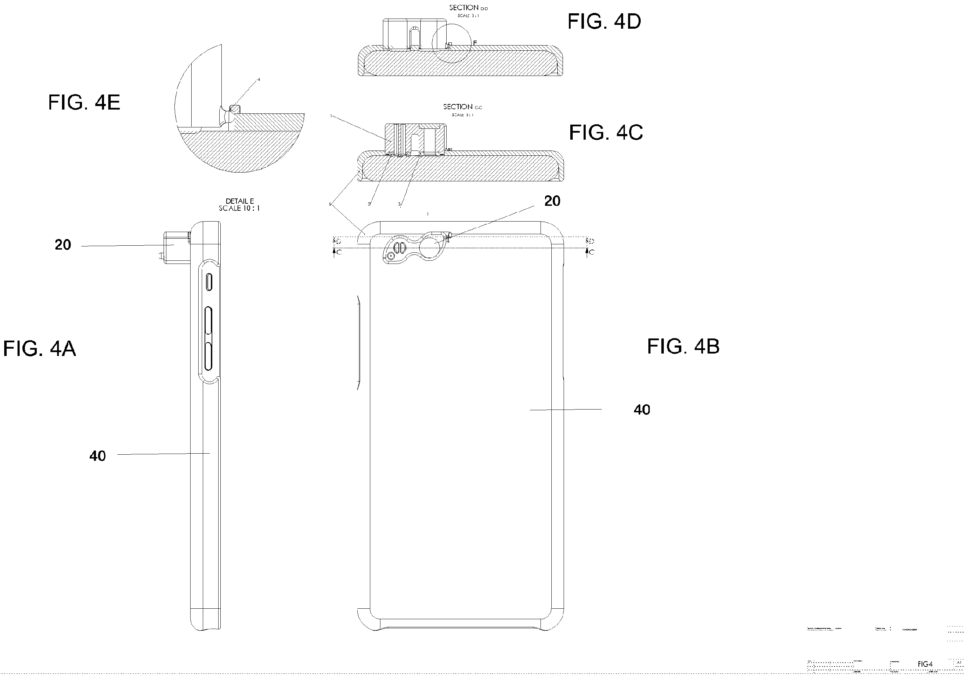

As seen in Figures 1 and 4A-4D, the invented system is based around hardware or a device 40, such as a smartphone, computer, iPod, digital camera, tablet, or other device. The device 40 provides a user interface, a light source, and an optical sensor. It also stores energy to provide power to these different components. In the preferred embodiment, the device is a smartphone such as the Nokia Lumia 1020, iPhone 5S, and the Samsung Galaxy 4, although tablets, personal computers, and other electronics will serve as well. In the preferred embodiments, the native flash of the camera is used as the source of light emission, although in other embodiments, the smartphone may control an external electronics module that emits light as well. The spectral distribution of the light source on the Nokia Lumia 1020, iPhone 5S, and the Samsung Galaxy 4 are shown in Figures 3A, 3B, and 3C, respectively. These Figure shows that both of the preferred 450 nm and 550 nm wavelengths are emitted by standard smartphone flashes. In preferred embodiments the camera of the smartphone is used as optical sensor, although the optical sensor may be ancillary sensors that evaluate light. Device 40 also contains a memory for storing software and data as well as a processor to execute the software. Additional sensors for detecting environmental data, such as ambient light may be included in the hardware as well.

The software 30 connects the various aspects of the invention and allows the user to interact with the controls as well as visualize the outputs of the analysis. The software controls the intensity, duration and timing sequence of the light source as well as the activation and parameters of the camera and/or sensor. It additionally can take inputs from additional sensors such as an ambient light sensor to be utilized in analysis. The software in some embodiments can analyze passive or active input from the camera to ensure the module is appropriately adhered to the hardware as well as appropriately contacting the skin substrate. The software further might prevent a calculated value to be obtained under certain constraints. In even further embodiments, it might provide visual, audible or tactile feedback to the user until the appropriate constraints are met. Once the software obtains data, it can utilize the input from the camera or sensor(s) to calculate concentrations of substances such as bilirubin within the cutaneous layer. Preferably, the software includes an algorithm that separates and analyzes the output from the return optical pathways to negate the effects of ambient light. Additionally the software could store these concentration values to provide a logged history to the user, patient, or caregivers, either directly or through wireless communications, to monitor changes and trending and to provide clinical recommendations to the patient and/or caregiver. In some embodiments, the software 30 may additionally upload and forward concentration values to a caregiver such as a doctor, nurse, or a hospital. This can be done automatically in real time or at regular intervals, or, only when requested or approved by the user possibly by way of a pop up window or side option. The software 30 may further provide reminders. For example, the software may alert a user if a reading was not been taken recently. Even further, in some embodiments, the software 30 will report device failure.

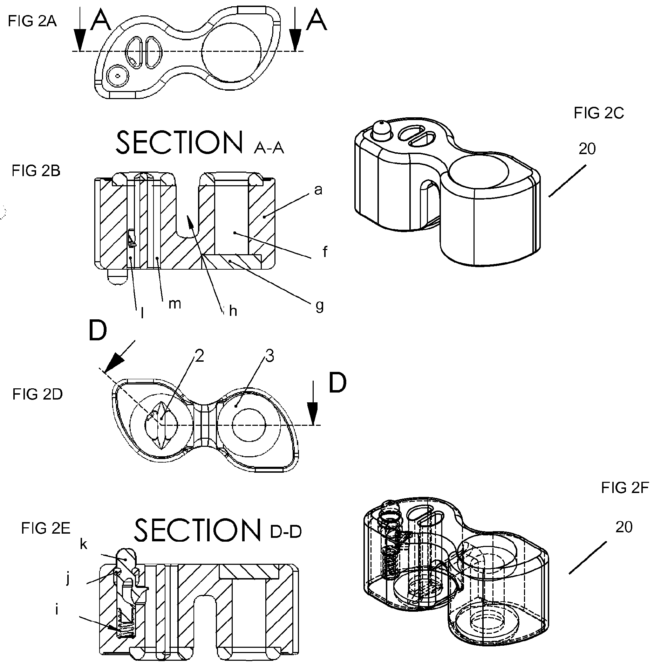

The invention also includes an ancillary module 20 as shown in Figure 1, 2A-2F, and 4A-4E. The ancillary module attaches to the light source and optical sensor to create a light-tight, non-transmissive barrier between these components and the patients skin. The ancillary module 20 may be attached by any known means including adhesives or interlocking parts. The light-tight feature is a vital feature of the present invention because it protects the integrity of the interface to the patient by ensuring it is appropriately in contact with the skin or other measurement substrate within acceptable pressure ranges to obtain the most accurate data. This function could be achieved through the compression of elastomeric elements 2b, 2c such as a rigid housing biased by spring loaded mechanical elements. Other possible light-tight barrier mechanisms can include inclined, ramped, or snap locking features, cases, pressure sensitive adhesives or other methods of attachment that would allow for sufficient compression of the light tight gasket(s) or seal. In some embodiments, the ancillary module further includes a mechanism for monitoring the quality of the seal. In some embodiments, the mechanism may be entirely electrical, such as a pressure sensitive touchscreen. In these embodiments, the mechanism will communicate with the software by way of the headphone jack or other input location. In other embodiments, it may be entirely mechanical. In Figure 2, a small spring 2i loaded light sealed 2j articulating toggle 2k reacts within the appropriate pressure range to remove a shutter blocking the lightpath to the camera, as further described below, such that the software shall recognize that the ancillary module 20 is properly sealed and ready for use. The toggle 2k is hinged within the housing allowing for rotational movement. Alternatively, it may be captured within a vertical cavity of the housing and could be held in place through retention features either in the housing or the toggle. In this embodiment, prior to applying the ancillary module 20 to the skin, the tip of the retention feature would extend distally from the housing. When the user begins to place the ancillary module 20 on the patient's skin, surface tension would result in a normal force being applied on the tip of the feature, exceeding the spring force of the feature, and causing it to recoil back into the housing. The spring force of the feature could be created by a plastic molded spring arm, a compression spring, a torsional spring or through other known force, proximity, transmittance or other sensor driven actuators. The articulation of this arm would then either insert or remove a portion of the arm either into or out of a path of light returning from the skin to the camera. This aberration or lack of aberration in the light could be sensed by the phone camera and recognized as an input to the software program. A similar mechanism could also be utilized to detect the appropriate attachment of the ancillary module to the hardware, which could also be achieved through image analysis as further described in the software section below or by utilizing other sensors or actuated aberrations

The ancillary module also acts as a housing to provide a light pathway 2f to enable the light source of the hardware to be directly transferred to the patient's skin. The ancillary module may contain one or more intermediate optical features such as a lens or high, low and bandpass filtering elements 2g. These options could allow light transmitted from the light source to be filtered to controlled wavelengths and transmitted with controlled losses in amplitude without interference from external sources due to the dimensional and optical characteristics of the housing components. The module could also have voids, gaps or additional light pathways or pipes 2h to allow sensors such as an ambient light sensor to have direct or indirect access to external light sources that would also influence the tissue properties and be able to be incorporated into software algorithms.

The module may further provide one or more return light pathways 21, 2m that allow light refracted within the skin to return to the camera sensor feature. For example, the invention might include multiple parallel return light paths that capture light from two or more different dimensional pathways. These pathways may vary in size and spacing to accommodate different devices and brands. This may be accomplished by a threaded connection between the camera section and the light source section with detends set for different devices as well as additional threaded adjustment(s) to adust the elevantion exis between the sections if desired. The several different pathways may direct light through various thicknesses of skin and allow the light to be transferred to the camera without the influence of other external sources due to the dimensional and optical characteristics of the housing components such as cavities or light pipes 2h.

The invention can be used in a multitude of embodiments, two of which are further described below. Although each embodiment is described through methods most optimal for that particular embodiment, the majority of the methods disclosed can be combined or used in parallel with other embodiments envisioned.

In the preferred embodiment the ancillary module is affixed to the device and is contacted to the skin. This is due to the fact that the alignment with the mobile device flash and camera is more critical than the alignment to the skin substrate within a jaundice patient population. The role of the interfaces could be reversed to instead adhere the ancillary module to the patient substrate or a particular target area of a substrate through a pressure sensitive adhesive patch. This would then require the hardware device to be connected to the module just during time of use, which could be accomplished through a similar light obscuring mechanism that is used on either side of the preferred embodiment or can be accomplished through other means of mechanical alignment and connection. In another embodiment, portions of the hardware utilized within the preferred embodiment could also be stored in a separate device or as part of the ancillary module to allow for additional filters or sensors not available on the hardware. The ancillary hardware could then be connected by various means of electrical connection such as through utilizing a stereo, dock or USB connection.

It will be understood by those of ordinary skill in the art that various changes may be made and equivalents may be substituted for elements without departing from the scope of the invention. In addition, many modifications may be made to adapt a particular feature or material to the teachings of the invention without departing from the scope thereof. Therefore, it is intended that the invention not be limited to the particular embodiments disclosed, but that the invention will include all embodiments falling within the scope of the claims.

## Claims

1. A system for monitoring the levels of at least one substance in skin based on the substance's optical properties, comprising:
a device including:
a memory;
a processor;
a camera integral to said device coupled to said processor;
a light source integral to said device coupled to said processor; and
a software module stored in the memory, said software module configured to run on said processor configured to control the light emitted by the light source, control the camera, and analyze at least one measurement of an amount of light from the camera to calculate concentrations of the at least one substance using an algorithm; and
an ancillary module (20) configured to couple to said device such that when the ancillary module is coupled to said device and placed on the skin the ancillary module creates a light-tight housing for the camera of said device.
wherein the ancillary module comprises one or more filters (2g) configured to control wavelengths of light transmitted by said light source or received by said camera, and
wherein the ancillary module includes at least one first light pathway (2f) configured to direct light from said light source to the skin and at least one second light pathway (2l, 2m)
configured to direct light reflected from the skin to said camera.

2. A system as claimed in claim 1 wherein the device is a mobile device being a smartphone, tablet, or computer.

3. A system as claimed in claim 1 wherein the ancillary module includes a sensor for measuring whether a light-tight seal has been created between said camera and the skin.

4. A system as claimed in claim 1 further comprising at least one ambient light sensor and wherein the software module is also configured to subtract a measurement of an amount of ambient light obtained from said ambient light sensor from said measurement of an amount of light measured by said camera to calculate an amount of reflected light introduced into the skin.

5. A system as claimed in claim 1 wherein the at least one substance is one or more of bilirubin, a bodily fluid, a body product, a drug marked for a a color marker, a drug containing a color marker, a biologic marked for a color marker property, and a biologic containing a a color marker.

6. A system as claimed in claim 1 wherein said calculation of a concentration of the at least one substance includes wavelength analysis of said at least one light measurement from said camera.

7. A system as claimed in claim 3 wherein said sensor is an electromechanical sensor, an electrical sensor, or a mechanical sensor.

8. A method for determining concentration levels of at least one substance in skin based on the substance's optical properties, comprising:
providing a device comprising:
a processor;
a memory coupled to said processor;
a light source integral to said device coupled to said processor;
a camera integral to said device coupled to said processor; and
a software module stored in the memory, said software module configured to run on said processor configured to control the light emitted by the light source, control the camera, and analyse at least one measurement of an amount of light from the camera to calculate concentrations of the at least one substance using an algorithm;;
providing an ancillary module (20) configured to couple to said device such that when the ancillary module (20) is coupled to said device and placed on the skin creates a light-tight housing for the camera of said device;
placing the said ancillary module (20) of the device on the surface of the skin;
activating, by said software module, the light source;
lighting the skin using the light source;
recording by said camera, a measurement of reflected light from the skin; and
calculating by said software module the concentration of the substance in the skin based on said measurement of reflected light from said camera,
wherein the ancillary module (20) comprises one or more filters (2g) configured to control wavelengths of light transmitted by said light source or received by said camera, and
wherein the ancillary module (20) includes at least one first light pathway (2f) configured to direct light from said light source to the skin and at least one second light pathway (2l, 2m) configured to direct light reflected from the skin to said camera

9. A method as claimed in claims 8 further including measuring, by a sensor of said ancillary module, whether a light-tight seal has been created between said camera and the skin..

10. A method as claimed in claim 8 wherein the device further comprises at least one ambient light sensor and further including:
measuring, by said ambient light sensor, an amount of ambient light obtained from said ambient light sensor;
subtracting, by said software module, said measurement of an amount of ambient light from said measurement of an amount of light measured by said camera to calculate an amount of reflected light introduced into the skin.

11. A method as claimed in claim 8 wherein the at least one substance is one or more of bilirubin, a bodily fluid, a body product, a drug marked for a color marker, a drug containing a a color marker, a biologic marked for a color marker p, and a biologic containing a color marker.

12. A method as claimed in claim 8 wherein said calculation of a concentration of the at least one substance includes wavelength analysis of said at least one light measurement from said camera.

## Patentansprüche

1. System zur Überwachung der Spiegel von mindestens einer Substanz in der Haut basierend auf den optischen Eigenschaften der Substanz, umfassend:
eine Vorrichtung, welche einschließt:
einen Speicher;
einen Prozessor;
eine Kamera, die integral mit der Vorrichtung ist, die an den Prozessor gekoppelt ist;
eine Lichtquelle, die integral mit der Vorrichtung ist, die an den Prozessor gekoppelt ist; und
ein Softwaremodul, das in dem Speicher gespeichert ist, wobei das Softwaremodul konfiguriert ist, um auf dem Prozessor zu laufen,
der konfiguriert ist, um das von der Lichtquelle emittierte Licht zu steuern, die Kamera zu steuern, und mindestens eine Messung einer Lichtmenge von der Kamera zu analysieren, um Konzentrationen der mindestens einen Substanz unter Verwendung eines Algorithmus zu berechnen; und
ein Hilfsmodul (20), das konfiguriert ist, um an die Vorrichtung zu koppeln, so dass das Hilfsmodul, wenn das Hilfsmodul an die Vorrichtung gekoppelt und auf der Haut platziert wird, ein lichtdichtes Gehäuse für die Kamera der Vorrichtung erzeugt,
wobei das Hilfsmodul ein oder mehrere Filter (2g) umfasst, die konfiguriert sind, um Wellenlängen des Lichts zu steuern, das durch die Lichtquelle gesendet oder von der Kamera empfangen wird, und wobei das Hilfsmodul mindestens einen ersten Lichtpfad (2f), der konfiguriert ist, um Licht von der Lichtquelle zu der Haut zu lenken, und mindestens einen zweiten Lichtpfad (21, 2m) einschließt, der konfiguriert ist, um von der Haut reflektiertes Licht zu der Kamera zu lenken.

2. System nach Anspruch 1, wobei die Vorrichtung eine mobile Vorrichtung ist, die ein Smartphone, Tablet oder Computer ist.

3. System nach Anspruch 1, wobei das Hilfsmodul einen Sensor einschließt, um zu messen, ob zwischen der Kamera und der Haut eine lichtdichte Versiegelung erzeugt worden ist.

4. System nach Anspruch 1, des Weiteren umfassend mindestens einen Umgebungslichtsensor, und wobei das Softwaremodul auch konfiguriert ist, um eine Messung einer Menge des Umgebungslichts, die von dem Umgebungslichtsensor erhalten wurde, von der Messung einer Lichtmenge abzuziehen, die von der Kamera gemessen wurde, um eine Menge des reflektierten Lichts zu berechnen, das in die Haut eingebracht wurde.

5. System nach Anspruch 1, wobei die mindestens eine Substanz eine oder mehrere von Bilirubin, einem Körperfluid, einem Körperprodukt, einem Arzneimittel, das durch einen Farbmarker markiert ist, einem Arzneimittel, das einen Farbmarker enthält, einem Biologikum, das durch eine Farbmarkereigenschaft markiert ist, und einem Biologikum, das einen Farbmarker enthält, ist.

6. System nach Anspruch 1, wobei die Berechnung einer Konzentration der mindestens einen Substanz eine Wellenlängenanalyse der mindestens einen Lichtmessung von der Kamera einschließt.

7. System nach Anspruch 3, wobei der Sensor ein elektromechanischer Sensor, ein elektrischer Sensor oder ein mechanischer Sensor ist.

8. Verfahren zum Ermitteln von Konzentrationsspiegeln von mindestens einer Substanz in der Haut basierend auf den optischen Eigenschaften der Substanz, umfassend:
Bereitstellen einer Vorrichtung, umfassend:
einen Prozessor;
einen an den Prozessor gekoppelten Speicher;
eine Lichtquelle, die integral mit der Vorrichtung ist, die an den Prozessor gekoppelt ist;
eine Kamera, die integral mit der Vorrichtung ist, die an den Prozessor gekoppelt ist; und
ein Softwaremodul, das in dem Speicher gespeichert ist, wobei das Softwaremodul konfiguriert ist, um auf dem Prozessor zu laufen, der konfiguriert ist, um das von der Lichtquelle emittierte Licht zu steuern, die Kamera zu steuern, und mindestens eine Messung einer Lichtmenge von der Kamera zu analysieren, um Konzentrationen der mindestens einen Substanz unter Verwendung eines Algorithmus zu berechnen;
Bereitstellen eines Hilfsmoduls (20), das konfiguriert ist, um an die Vorrichtung zu koppeln, so dass, wenn das Hilfsmodul (20) an die Vorrichtung gekoppelt ist und auf der Haut platziert wird, es ein lichtdichtes Gehäuse für die Kamera der Vorrichtung erzeugt;
Platzieren des Hilfsmoduls (20) der Vorrichtung auf der Oberfläche der Haut;
Aktivieren der Lichtquelle durch das Softwaremodul;
Beleuchten der Haut unter Verwendung der Lichtquelle;
Aufzeichnen einer Messung des von der Haut reflektierten Lichts durch die Kamera; und
Berechnen der Konzentration der Substanz in der Haut basierend auf der Messung des reflektierten Lichts von der Kamera durch das Softwaremodul,
wobei das Hilfsmodul (20) ein oder mehrere Filter (2g) umfasst, die konfiguriert sind, um Wellenlängen des Lichts zu steuern, das durch die Lichtquelle gesendet oder von der Kamera empfangen wird, und
wobei das Hilfsmodul (20) mindestens einen ersten Lichtpfad (2f), der konfiguriert ist, um Licht von der Lichtquelle zu der Haut zu lenken, und mindestens einen zweiten Lichtpfad (21, 2m) einschließt, der konfiguriert ist, um von der Haut reflektiertes Licht zu der Kamera zu lenken.

9. Verfahren nach Anspruch 8, des Weiteren einschließend Messen durch einen Sensor des Hilfsmoduls, ob zwischen der Kamera und der Haut eine lichtdichte Versiegelung erzeugt worden ist.

10. Verfahren nach Anspruch 8, wobei die Vorrichtung des Weiteren mindestens einen Umgebungslichtsensor umfasst und des Weiteren einschließt:
Messen einer Menge an Umgebungslicht, die von dem Umgebungslichtsensor erhalten wird, durch den Umgebungslichtsensor;
Abziehen der Messung einer Menge an Umgebungslicht von der Messung einer Lichtmenge, die durch die Kamera gemessen wurde, durch das Softwaremodul, um eine Menge an reflektiertem Licht zu berechnen, das in die Haut eingebracht wurde.

11. Verfahren nach Anspruch 8, wobei die mindestens eine Substanz eine oder mehrere von Bilirubin, einem Körperfluid, einem Körperprodukt, einem Arzneimittel, das durch einen Farbmarker markiert ist, einem Arzneimittel, das einen Farbmarker enthält, einem Biologikum, das durch einen Farbmarker p markiert ist, und einem Biologikum, das einen Farbmarker enthält, ist.

12. Verfahren nach Anspruch 8, wobei die Berechnung einer Konzentration der mindestens einen Substanz eine Wellenlängenanalyse der mindestens einen Lichtmessung von der Kamera einschließt.

## Revendications

1. Système de surveillance des niveaux d'au moins une substance dans la peau sur la base des propriétés optiques de la substance, comprenant :
un dispositif comprenant :
une mémoire ;
un processeur ;
une caméra intégrée audit dispositif couplée audit processeur ;
une source de lumière intégrée audit dispositif couplée audit processeur ; et
un module logiciel stocké dans la mémoire, ledit module logiciel étant configuré pour fonctionner sur ledit processeur configuré pour contrôler la lumière émise par la source de lumière, contrôler la caméra, et analyser au moins une mesure d'une quantité de lumière provenant de la caméra pour calculer des concentrations de l'au moins une substance en utilisant un algorithme ; et
un module auxiliaire (20) configuré pour se coupler audit dispositif de telle sorte que lorsque le module auxiliaire est couplé audit dispositif et placé sur la peau, le module auxiliaire crée un logement étanche à la lumière pour la caméra dudit dispositif,
le module auxiliaire comprenant un ou plusieurs filtres (2g) configurés pour contrôler les longueurs d'onde de la lumière transmise par ladite source de lumière ou reçue par ladite caméra, et
le module auxiliaire comprenant au moins un premier trajet de lumière (2f) configuré pour diriger la lumière de ladite source de lumière vers la peau et au moins un deuxième trajet de lumière (21, 2m) configuré pour diriger la lumière réfléchie par la peau vers ladite caméra.

2. Système selon la revendication 1, le dispositif étant un dispositif mobile qui est un smartphone, une tablette ou un ordinateur.

3. Système selon la revendication 1, le module auxiliaire comprenant un capteur pour mesurer si un joint étanche à la lumière a été créé entre ladite caméra et la peau.

4. Système selon la revendication 1, comprenant en outre au moins un capteur de lumière ambiante et le module logiciel étant également configuré pour soustraire une mesure d'une quantité de lumière ambiante obtenue à partir dudit capteur de lumière ambiante de ladite mesure d'une quantité de lumière mesurée par ladite caméra pour calculer une quantité de lumière réfléchie introduite dans la peau.

5. Système selon la revendication 1, l'au moins une substance étant une ou plusieurs substances parmi la bilirubine, un fluide corporel, un produit corporel, un médicament marqué pour un marqueur de couleur, un médicament contenant un marqueur de couleur, un produit biologique marqué pour une propriété de marqueur de couleur, et un produit biologique contenant un marqueur de couleur.

6. Système selon la revendication 1, ledit calcul d'une concentration de l'au moins une substance comprenant l'analyse de la longueur d'onde de ladite au moins une mesure de lumière provenant de ladite caméra.

7. Système selon la revendication 3, ledit capteur étant un capteur électromécanique, un capteur électrique ou un capteur mécanique.

8. Procédé pour déterminer les niveaux de concentration d'au moins une substance dans la peau sur la base des propriétés optiques de la substance, comprenant :
la fourniture d'un dispositif comprenant :
un processeur ;
une mémoire couplée audit processeur ;
une source de lumière intégrée audit dispositif couplée audit processeur ;
une caméra intégrée audit dispositif couplée audit processeur ; et
un module logiciel stocké dans la mémoire, ledit module logiciel étant configuré pour fonctionner sur ledit processeur configuré pour contrôler la lumière émise par la source de lumière, contrôler la caméra, et analyser au moins une mesure d'une quantité de lumière provenant de la caméra pour calculer des concentrations de l'au moins une substance en utilisant un algorithme ;
la fourniture d'un module auxiliaire (20) configuré pour se coupler audit dispositif de sorte que lorsque le module auxiliaire (20) est couplé audit dispositif et placé sur la peau, il crée un logement étanche à la lumière pour la caméra dudit dispositif ;
le placement dudit module auxiliaire (20) du dispositif sur la surface de la peau ;
l'activation, par ledit module logiciel, de la source de lumière ;
l'éclairage de la peau à l'aide de la source de lumière ;
l'enregistrement, par ladite caméra, d'une mesure de la lumière réfléchie par la peau ; et
le calcul par ledit module logiciel de la concentration de la substance dans la peau sur la base de ladite mesure de la lumière réfléchie par ladite caméra,
le module auxiliaire (20) comprenant un ou plusieurs filtres (2g) configurés pour contrôler les longueurs d'onde de la lumière transmise par ladite source de lumière ou reçue par ladite caméra, et
le module auxiliaire (20) comprenant au moins un premier trajet de lumière (2f) configuré pour diriger la lumière de ladite source de lumière vers la peau et au moins un deuxième trajet de lumière (21, 2m) configuré pour diriger la lumière réfléchie par la peau vers ladite caméra.

9. Procédé selon la revendication 8, comprenant en outre la mesure, par un capteur dudit module auxiliaire, de la création d'un joint étanche à la lumière entre ladite caméra et la peau.

10. Procédé selon la revendication 8, le dispositif comprenant en outre au moins un capteur de lumière ambiante, et comprenant en outre :
la mesure, par ledit capteur de lumière ambiante, d'une quantité de lumière ambiante obtenue à partir dudit capteur de lumière ambiante ;
la soustraction, par ledit module logiciel, de ladite mesure d'une quantité de lumière ambiante de ladite mesure d'une quantité de lumière mesurée par ladite caméra pour calculer une quantité de lumière réfléchie introduite dans la peau.

11. Procédé selon la revendication 8, l'au moins une substance étant une ou plusieurs substances parmi la bilirubine, un fluide corporel, un produit corporel, un médicament marqué pour un marqueur de couleur, un médicament contenant un marqueur de couleur, un produit biologique marqué pour un marqueur de couleur p, et un produit biologique contenant un marqueur de couleur.

12. Procédé selon la revendication 8, ledit calcul d'une concentration de l'au moins une substance comprenant l'analyse de la longueur d'onde de ladite au moins une mesure de lumière provenant de ladite caméra.
